# EUROPEAN PATENT SPECIFICATION

(11) **EP 3 069 723 B1**
(45) Date of publication and mention of the grant of the patent: **18.04.2018**
(21) Application number: 15842769.0
(22) Date of filing: 28.08.2015
(51) Int. Cl.: A61K 31/7048, A61K 31/495, A61K 9/20, A61K 9/48, A61P 11/14, A61P 11/10, A61P 11/06

(54) **NARINGIN AND LEVOCETIRIZINE HYDROCHLORIDE PHARMACEUTICAL COMPOSITION AND PREPARATION THEREOF**
PHARMAZEUTISCHE ZUSAMMENSETZUNG AUS NARINGIN UND LEVOCETIRIZIN-HYDROCHLORID SOWIE HERSTELLUNG DAVON
COMPOSITION PHARMACEUTIQUE À BASE DE NARINGINE ET DE CHLORHYDRATE DE LÉVOCÉTIRIZINE, ET PRÉPARATION DE CELLE-CI

(30) Priority: 18.09.2014 CN 201410479766
(43) Date of publication of application: 21.09.2016
(73) Proprietor: Sun Yat-Sen University, Guangzhou, Guangdong 510275 (CN)
(72) Inventor: SU, Weiwei, Guangzhou Guangdong 510000 (CN); JIAO, Haoyan, Guangzhou Guangdong 510000 (CN); LIAO, Yan, Guangzhou Guangdong 510000 (CN); LI, Peibo, Guangzhou Guangdong 510000 (CN); PENG, Wei, Guangzhou Guangdong 510000 (CN); WANG, Yonggang, Guangzhou Guangdong 510000 (CN)
(74) Representative: Cabinet Bleger-Rhein-Poupon
(86) International application number: PCT/CN2015/088434
(87) International publication number: WO 2016/041439

(56) References cited:
- CN-A- 1 430 967
- CN-A- 103 830 208
- CN-A- 104 224 819
- SCHOEPKE, N ET AL.: 'The Inhibition by Levocetirizine and Fexofenadine of the Histamine-induced Wheal and Flare Response in Healthy Caucasian and Japanese Volunteers' ACTA DERM VENEREOL 31 December 2013, pages 286 - 293, XP055327925

## Description

### Technical Field

The present invention relates to a pharmaceutical composition of naringin for relieving cough, reducing sputum and relieving asthma, and preparations thereof.

### Background

Cough and expectoration are two common symptoms of respiratory diseases, which are closely correlated with each other in pathology. Generally, cough is accompanied by expectoration, and sputum production often causes cough. Prolonged course of disease may lead to emphysema, bronchiectasis, pulmonary heart disease and so on. Cough variant asthma refers to a special type of asthma with chronic cough as the main or sole clinical manifestation.

At present, the most widely used cough relieving drugs include codeine phosphate, dextromethorphan hydrobromide and others.

Codeine phosphate is a chemical drug acting on central nervous system that is widely used for relieving cough or common cold, but suffers from elevated supervisory levels again and again by Food & Drug Administration due to its serious adverse effects in recent years. The common adverse effects include psychological abnormity or illusions; weak, slow or irregular respiration; fast and slow heart rate; some extremely uncommon adverse effects, including convulsion, tinnitus, tremor or uncontrollable muscle movements, and others; urticaria; itching, rash or swollen face, and other allergic reactions; mental depression, and muscle rigidity etc. The long-term administration may lead to dependence. The tendency to dependence at a usual dose is weaker than that of other morphine-like drugs. The typical symptoms include goose flesh, anorexia, diarrhea, toothache, nausea and vomiting, runny nose, shaking chills, sneezing, yawning, sleep disorders, gastrospasm, excessive sweating, weakness and fatigue, increased heart rate, emotion or fever of unknown causes.

Dextromethorphan hydrobromide is also a common chemical drug acting on central nervous system that is used for relieving cough, and may be available from a pharmacy by the consumer. However, serious adverse effects occur with increasing dosage, especially in the case of drug abuse. It has repeatedly reported abroad that over dosing of capsules filled with dextromethorphan powder leads to the death of patients. The U.S. Food & Drug Administration persistently pays attention to dextromethorphan abuse, and issues a warning of forbidding dextromethorphan abuse. The U.S. Food & Drug Administration suggests that proper administration of dextromethorphan at a low dose can safely and effectively inhibit the symptoms of common cold, and dextromethorphan abuse may lead to death and other serious adverse effects, such as brain injury, epileptic seizure, loss of consciousness and irregular heart beat.

Naringin has a good cough relieving, sputum reducing and asthma relieving effect, has no drug dependence, and has a minimal adverse effect. CN1430967 discloses the use of naringin for the treatment of cough. CN103830208 discloses the use of anti-histamines including levocetirizine for the treatment of asthma. Therefore, development of compound drugs of naringin having better curative effect is of promising prospect in medicine.

### Summary of the Invention

The present invention provides a pharmaceutical composition of naringin for relieving cough, reducing sputum and relieving asthma, and preparations thereof.

The pharmaceutical composition comprises naringin and levocetirizine hydrochloride.

The preferred daily dose of the composition comprises 27.5-275 mg of naringin and 1.25-12.5 mg of levocetirizine hydrochloride.

The preferred weight ratio of naringin to levocetirizine hydrochloride in the pharmaceutical composition is recommended to be 20:1, and the preferred daily dose of the pharmaceutical composition contains 40 mg of naringin and 2 mg of levocetirizine hydrochloride per unit preparation.

The pharmaceutical composition may be prepared into a pharmaceutically acceptable tablet, aqueous solution, capsule, aerosol, and so on. The adjuvant in the present pharmaceutical composition may include starch, lactose, mannitol, calcium hydrophosphate, carboxymethyl starch or a salt or a substituted derivate thereof, dextrin, chitosan, polyvinyl pyrrolidone, cellulose or a derivate thereof, or polyethylene glycol.

It is confirmed through test that the ingredients naringin and levocetirizine hydrochloride in the pharmaceutical composition of the present invention have a synergistic effect. The efficacy of the pharmaceutical composition is obviously superior to that of naringin or levocetirizine hydrochloride that is used alone, and exhibits a good cough relieving, sputum reducing and asthma relieving effect. The pharmaceutical composition of the present invention is useful in the treatment of cough, expectoration, and wheezing caused by cough variant asthma, and causes no adverse effects such as somnolence, nausea, emesis, and vomiting after administration. The pharmaceutical composition may be added with conventional adjuvents and prepared through any conventional process into drugs for relieving cough, reducing sputum and relieving asthma.

The effect of naringin in combination with other drugs is also investigated by the inventors. The results show that no synergistic effect is exhibited when naringin is used respectively in combination with dimenhydrinate hydrochloride, theohydramine hydrochloride and chlorpheniramine maleate, loratadine, desloratadine, azelastine hydrochloride, mizolastine, and epinastine hydrochloride.

### Detailed Description

The present invention is further described below by way of examples.

### Example 1:

### Inhibition on citric acid induced cough in guinea pig

### 1. Materials

1.1 Test animals: qualified Hartley guinea pigs, weight 250-300 g, female : male 1:1, available from Guangdong Medical Laboratory Animal Center.
1.2 Drugs and reagents: robitussin; naringin, formulated at a dosage of 120 mg per person per day; levocetirizine hydrochloride, formulated at a dosage of 6 mg per person per day; Composition (1), formulated at a dosage of 27.5 mg naringin and 1.25 mg levocetirizine hydrochloride per person per day; Composition (2), formulated at a dosage of 27.5 mg naringin and 12.5 mg levocetirizine hydrochloride per person per day; Composition (3), formulated at a dosage of 275 mg naringin and 1.25 mg levocetirizine hydrochloride per person per day; Composition (4), formulated at a dosage of 275 mg naringin and 12.5 mg levocetirizine hydrochloride per person per day; and Composition (5), formulated at a dosage of 120 mg naringin and 6 mg levocetirizine hydrochloride per person per day.
1.3 Instrument: YLS-8A cough and asthma inducing instrument (a product available from Equipment Station of Shandong Academy of Medical Sciences).

### 2. Methods

72 qualified Hartley guinea pigs weighed 250-300 g were randomly assigned to 9 groups including a blank control group, a naringin group, a robitussin group, a levocetirizine hydrochloride group, and Composition (1) to (5) groups, each group having 8 animals. The guinea pigs in each group were administered by oral gavage at a dosage of 0.5 ml/100 g of body weight, and equal volume of saline was given to the animals in the blank control group. 1 h after administration by oral gavage, the animals received spray of citric acid for 7 min. After the spray was completed, the cough counts were observed and recorded in ten minutes from the start of the spray (the typical cough is loud and clear and often accompanied by a forward rush).

### 3. Results

As can be seen from Table 1, when administered alone, both naringin and levocetirizine hydrochloride have an obvious cough relieving effect (P<0.05 or 0.01, compared with the blank group). Each combination of naringin with levocetirizine hydrochloride also has a good cough relieving effect that is obviously superior to that of naringin or levocetirizine hydrochloride when administered alone, and the difference is of statistical significance (P<0.05 or 0.01, compared with the groups given with naringin or levocetirizine hydrochloride alone). The results show that the pharmaceutical composition has a good cough relieving effect that is obviously superior to that of naringin or levocetirizine hydrochloride when administered alone.

**Table 1. Inhibition of test drugs on citric acid induced cough (n=8)**

| Group | Cough count |
|---|---|
| Blank control group | 35.5±5.9 |
| Robitussin group | 24.6±6.8* |
| Naringin group | 22.4±3.7** |
| Levocetirizine hydrochloride group | 26.3±7.1* |
| Composition (1) group | 15.6±5.8** 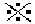 □ 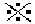 |
| Composition (2) group | 14.2±6.3**□□ |
| Composition (3) group | 12.7±3.3** 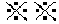 □□ |
| Composition (4) group | 11.8±6.4** 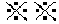 □□ 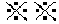 |
| Composition (5) group | 11.1±4.5**□□ |

| | |
|---|---|
| Note: 1. Compared with the blank control group, *P<0.05, **P<0.01; 2. Compared with the naringin group, 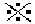 P<0.05, 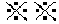 P<0.01; 3. Compared with the levocetirizine hydrochloride group, ^{□}P<0.05, ^{□□}P<0.01. | |

### Example 2:

### Effect on excretion of phenol red in mice

### 1. Materials

1.1 Test animals: Kunming mice, female : male 1:1, weight 30-40 g, available from Guangdong Medical Laboratory Animal Center.
1.2 Drugs and reagents: ambroxol; naringin, formulated at a dosage of 120 mg per person per day; levocetirizine hydrochloride, formulated at a dosage of 6 mg per person per day; Composition (1), formulated at a dosage of 27.5 mg naringin and 1.25 mg levocetirizine hydrochloride per person per day; Composition (2), formulated at a dosage of 27.5 mg naringin and 12.5 mg levocetirizine hydrochloride per person per day; Composition (3), formulated at a dosage of 275 mg naringin and 1.25 mg levocetirizine hydrochloride per person per day; Composition (4), formulated at a dosage of 275 mg naringin and 12.5 mg levocetirizine hydrochloride per person per day; and Composition (5), formulated at a dosage of 120 mg naringin and 6 mg levocetirizine hydrochloride per person per day.
1.3 Instrument: Hitachi 3010 UV and visible spectrophotometer.

### 2. Methods

The Kunming mice (female : male 1:1) were randomly assigned to a blank control group, an ambroxol group, a naringin group, a levocetirizine hydrochloride group, and Composition (1) to (5) groups, each group having 10 animals. The mice were administered by oral gavage at a dosage of 2 ml/10 g for consecutive 2 days. 30 min after the last dose, 5% phenol red in saline was intraperitoneally injected at a dosage of 0.2 ml/10 g. After 30 min, the mice were sacrificed, and the trachea was detached. A section of the trachea from the thyroid cartilage to a branch of the trachea was excised, and placed in a test tube containing 3 ml of saline, to which 0.1 ml of a 15% sodium bicarbonate solution was then added. After centrifugation, the supernatant was taken and measured for the OD value at 546 nm. The phenol red content was calculated from a phenol red standard curve. For constructing the standard curve, 0.1 µg/ml, 0.3 µg/ml, 0.5 µg/ml, 0.7 µg/ml, 1 µg/ml, 3 µg/ml, 5 µg/ml, and 10 µg/ml standard phenol red solutions were formulated respectively. If the drug can improve the secretory function of the respiratory tract, the excretion of phenol red can be enhanced.

### Results:

As can be seen from Table 2, when administered alone, both naringin and levocetirizine hydrochloride can obviously enhance the excretion of phenol red in mice (P<0.05 or 0.01, compared with the blank group), thus having a significant sputum reducing effect. Each combination of naringin with levocetirizine hydrochloride also has a good effect on enhancement of the excretion of phenol red in mice that is obviously superior to that of naringin or levocetirizine hydrochloride when administered alone, and the difference is of statistical significance (P<0.05 or 0.01, compared with the groups given with naringin or levocetirizine hydrochloride alone). The results show that the combination of naringin with levocetirizine hydrochloride has a good sputum reducing effect that is obviously superior to that of naringin or levocetirizine hydrochloride when administered alone.

**Table 2. Effect of test drugs on excretion of phenol red in mice (n=10)**

| Group | Phenol red concentration (µg/ml) |
|---|---|
| Blank control group | 0.65±0.25 |
| Ambroxol group | 1.55±0.46** |
| Naringin group | 1.64±0.67** |
| Levocetirizine hydrochloride group | 1.13±0.64* |
| Composition (1) group | 2.18±0.68** 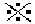 □ |
| Composition (2) group | 2.13±0.55** 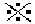 □ 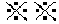 |
| Composition (3) group | 2.44±0.62**□□ 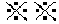 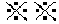 |
| Composition (4) group | 2.51±0.77**□□ |
| Composition (5) group | 2.56±0.35**□□ |

| | |
|---|---|
| Note: 1. Compared with the blank control group, *P<0.05, **P<0.01; 2. Compared with the naringin group, 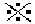 P<0.05, 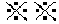 P<0.01; 3. Compared with the levocetirizine hydrochloride group, ^{□}P<0.05, ^{□□}P<0.01. | |

### Example 3:

Effect on ovalbumin induced atopic cough (cough variant asthma)

### 1. Materials

1.1 Test animals: Hartley guinea pig, male, weight 250-300 g, SPF grade, available from Guangdong Medical Laboratory Animal Center.
1.2 Drugs and reagents: cyclophosphamide; ovalbumin; capsaicin; acemecholine; cofetol cough syrup (compound codeine phosphate solution); naringin, formulated at a dosage of 120 mg per person per day; levocetirizine hydrochloride, formulated at a dosage of 6 mg per person per day; Composition (1), formulated at a dosage of 27.5 mg naringin and 1.25 mg levocetirizine hydrochloride per person per day; Composition (2), formulated at a dosage of 27.5 mg naringin and 12.5 mg levocetirizine hydrochloride per person per day; Composition (3), formulated at a dosage of 275 mg naringin and 1.25 mg levocetirizine hydrochloride per person per day; formulated at a dosage of 275 mg naringin and 12.5 mg levocetirizine hydrochloride per person per day; and Composition (5), formulated at a dosage of 120 mg naringin and 6 mg levocetirizine hydrochloride per person per day.
1.3 Instrument: BUXCO cough system and whole body plethysmograph (available from BUXCO Inc. (US)).

### 2. Methods

2.1 Grouping: male Hartley guinea pigs weighed 250-300 g were randomly assigned to a normal control group, a model control group, a cofetol group, a naringin group, a levocetirizine hydrochloride group, and Composition (1) to (5) groups, each group having 10 animals.
2.2 Modeling: except for the normal control group, the guinea pigs in the remaining groups were challenged by intraperitoneally injecting cyclophosphamide at a dosage of 30 mg/kg at day 1, intraperitoneally injecting 1 mL of a suspension containing 2 mg ovalbumin and 100 mg aluminium hydroxide at day 3, and injecting 1 mL of a suspension containing 0.01 mg ovalbumin and 100 mg aluminium hydroxide after 3 weeks. The guinea pigs in the normal control group were intraperitoneally injected with 1 mL of saline. After 3 weeks, all the animals for modeling were challenged by nebulization of a 1% ovalbumin solution for 90 s, and the animals in the normal control group inhaled saline for 90 s by nebulization.
2.3 Administration: 24 hrs after challenge, the animals in each group were administered according to the dosages give in 1.2 (Example 3), for consecutive 7 days. The cough of guinea pig were counted as follows. 1 hr after the last dose, the guinea pigs were placed in a Buxco cough recorder, and induced to cough by using 1 mL of 50 µmol/L capsaicin, and the cough counts in 10 min (including the nebulization time) were recorded.
2.4 Determination of airway responsiveness (AR) in guinea pig: 24 hrs after cough determination, the enhanced pause (Penh) of guinea pig was determined in the Buxco whole body plethysmograph, and the variation in Penh after challenge by nebulization of acemecholine (MeCh) was measured.

The acemecholine (MeCh) concentrations used for challenging were respectively, from low to high, 100 mg/L, 200 mg/L, 400 mg/L, 800 mg/L, and 1600 mg/L. The average Penh upon challenge with each level of MeCh was recorded, and converted into percentages (Penh%) relative to the Penh value upon challenge with normal saline (NS), which was used as an evaluation criterion for AR.
2.5 Bronchoalveolar lavage and differential leukocyte counts in bronchial alveolar lavage fluid (BALF): After AR determination, the guinea pigs were anesthetized with 30 mg/kg of pentobarbital sodium. Then, the neck skin was cut, and a median incision was made on the trachea, in which a tracheal cannula was inserted. Bronchoalveolar lavage was performed with 5 mL of saline. This was repeated 3 times, and the bronchial alveolar lavage fluid was collected. All the bronchial alveolar lavage fluid was centrifuged at 4°C and 1500 rpm for 10 min, and the supernatant was stored at -80°C for later use.
2.6 Histological section of lung: part of the right lung tissue was frozen sliced, conventionally immobilized, dehydrated, and stained with HE, to observe the histopathological change in the airway and lung tissue.

### 3. Results

### 3.1 Cough count

As can be seen from Table 3, the cough counts in the model group is obviously increased (P<0.01) compared with the normal control group. After dosing, the cough counts in each treatment group are reduced, and of statistical difference, compared with the model control group. The cough counts in each of the groups treated with the compositions of naringin and levocetirizine hydrochloride are considerably reduced and of statistical difference (P<0.05 or 0.01) compared with the group administered with naringin or levocetirizine hydrochloride alone. The results suggest that the pharmaceutical composition has a good cough relieving effect for the ovalbumin induced cough that is obviously superior to that of naringin or levocetirizine hydrochloride when administered alone.

**Table 3. Effect of test drugs on capsaicin induced cough in guinea pigs (n=10)**

| Groups | Cough counts |
|---|---|
| Normal control group | 14.3 ± 4.5 |
| Model control group | 25.5 ± 3.9^{##} 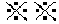 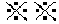 |
| Cofetol group | 15.7 ± 5.7* 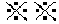 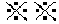 |
| Naringin group | 12.9 ± 4.3** |
| Levocetirizine hydrochloride group | 16.5 ± 7.8* |
| Composition (1) group | 8.2 ± 3.8**□□ |
| Composition (2) group | 7.6 ± 4.1**□□ |
| Composition (3) group | 5.1 ± 1.3**□□ |
| Composition (4) group | 4.2 ± 2.9**□□ |
| Composition (5) group | 4.2 ± 0.8**□□ |

| | |
|---|---|
| Note: 1. Compared with the normal control group, ^{##}P<0.01; 2. Compared with the model control group, *P<0.05, **P<0.01; 3. Compared with the naringin group, 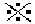 P<0.05, 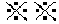 P<0.01; 4. Compared with the levocetirizine hydrochloride group, ^{□}P<0.05, ^{□□}P<0.01. | |

### 3.2 Airway responsiveness

As can be seen from Table 4, the airway responsiveness in the model group is obviously increased (P<0.01) compared with the normal control group. After dosing, the airway responsiveness in each treatment group was reduced. The reduction in acemecholine (MeCh) induced airway hyperresponsiveness in each of the groups treated with the compositions of naringin and levocetirizine hydrochloride is higher than that in the group administered with naringin or levocetirizine hydrochloride alone (P<0.05 or 0.01, compared with the groups administered with a single agent). The results suggest that the pharmaceutical composition of naringin and levocetirizine hydrochloride has a notable asthma relieving effect for ovalbumin induced cough variant asthma that is obviously superior to that of naringin or levocetirizine hydrochloride when administered alone.

**Table 4. Effect of test drugs on airway responsiveness in guinea pigs (n=10)**

| Group | Airway responsiveness under challenge with different concentrations of MeCh | | | | |
|---|---|---|---|---|---|
| | 100 mg/L | 200 mg/L | 400 mg/L | 800 mg/L | 1600 mg/L |
| Normal control group | 118±25 | 285±36 | 558±136 | 1117±254 | 1824±523 |
| Model control group | 625±154^{##} | 1754±543^{##} | 2876±867^{##} | 6421±1426^{##} | 11529±3579^{##} |
| Cofetol group | 225±41** | 793±33** | 946±82** | 1987±254** | 2966±217** |
| Naringin group | 276±69** | 783±147** | 857±108** | 1636±169** | 2749±253** |
| Levocetirizine hydrochloride group | 256±34** | 765±172** | 832±143** | 1512±204** | 2359±477** |
| Composition (1) group | 239±46** | 467±54** 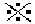 □ | 556±56**□□ 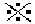 | 1325±105** 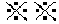 □□ | 2022±331** □ |
| Composition (2) group | 257±68** | 448±69** 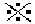 □ | 509±102**□□ 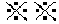 | 1189±168** 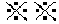 □□ | 1526±267** □□ |
| Composition (3) group | 153±39**□□ | 399±44* 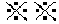 *□□ | 439±97** 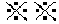 □□ | 813±247** 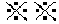 □□ | 1466±211** □□ |
| Composition (4) group | 86±36**□□ | 268±28** 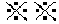 □□ | 355±103** 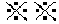 □□ | 655±139** 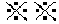 □□ | 1263±89** □□ |
| Composition (5) group | 98±15**□□ | 259±27** 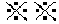 □□ | 368±91** 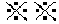 □□ | 601±157** 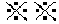 □□ | 1391±214** □□ |

| | | | | | |
|---|---|---|---|---|---|
| Note: 1. Compared with the normal control group, ^{#}P<0.05, ^{##}P<0.01; 2. Compared with the model control group, *P<0.05, **P<0.01; 3. Compared with the naringin group, 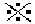 P<0.05, 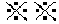 P<0.01; 4. Compared with the levocetirizine hydrochloride group, ^{□}P<0.05, ^{□□}P<0.01. | | | | | |

As can be seen from Table 5, the total leukocyte counts, and the total lymphocyte, neutrophil, and eosinophil counts in the model control group are obviously increased (P<0.01) compared with the normal control group. When administered alone, naringin and levocetirizine hydrochloride can also significantly reduce the total leukocyte counts, and the total lymphocyte, neutrophil, and eosinophil counts (P<0.05 or 0.01 compared with the model group). Each of the compositions of naringin and levocetirizine hydrochloride can significantly reduce the total leukocyte counts, and the total lymphocyte, neutrophil, and eosinophil counts (P<0.05 or 0.01 compared with the model group), and the effect on reduction of the total leukocyte counts, and the total lymphocyte, neutrophil, and eosinophil counts are greatly higher than that of naringin or levocetirizine hydrochloride when administered alone (P<0.05 or 0.01 compared with the group administered with a single agent. The results suggest that the pharmaceutical composition is advantageous over naringin or levocetirizine hydrochloride administered alone in the inhibition of inflammatory cells.

**Table 5. Results of differential leukocyte counts in bronchial alveolar lavage fluid from guinea pigs after administration with test drugs (n=10)**

| Group | Total leukocyte counts (10⁷/L) | Neutrophil counts (10⁷/L) | Lymphocyte counts (10⁷/L) | Eosinophil counts (107/L) |
|---|---|---|---|---|
| Normal control group | 49.3±14.6 | 3.4±2.1 | 3.1±1.0 | 4.2±1.3 |
| Model control group | 192.6±51.7^{##} | 192.6±51.7^{##} | 192.6±51.7^{##} | 192.6±51.7^{##} |
| Cofetol group | 155.3±32.4* | 35.4±14.3* | 25.6±6.9** | 34.5±8.8* |
| Naringin group | 135.5±28.6** | 30.1±6.5** | 21.5±3.7** | 27.9±5.6** |
| Levocetirizine hydrochloride group | 156.4±31.8* | 40.6±26.8* | 24.9±6.7** | 30.2±8.7* |
| Composition (1) group | 90.5±24.1**□□ | 14.5±3.8**□□ 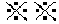 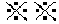 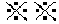 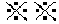 | 15.6±8.7**□ 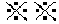 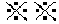 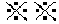 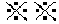 | 18.3±6.9**□□ |
| Composition (2) group | 86.3±25.1**□□ | 17.3±6.9**□□ | 14.2±6.4**□□ | 19.5±2.6**□□ |
| Composition (3) group | 81.7±34.5**□□ | 10.8±2.1**□□ | 12.5±3.4**□□ | 10.5±3.9**□□ |
| Composition (4) group | 66.8±19.8**□□ | 9.5±4.3**□□ | 7.6±3.1**□□ | 6.9±1.8**□□ |
| Composition (5) group | 61.2±13.7**□□ | 6.4±2.7**□□ | 8.4±2.5**□□ | 6.5±1.0**□□ |

| | | | | |
|---|---|---|---|---|
| Note: 1. Compared with the normal control group, ^{#}P<0.05, ^{##}P<0.01; 2. Compared with the model control group, *P<0.05, **P<0.01; 3. Compared with the naringin group, 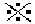 P<0.05, 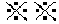 P<0.01; 4. Compared with the levocetirizine hydrochloride group, ^{□}P<0.05, ^{□□}P<0.01. | | | | |

### 3.4 Histological section results of lung

After dosing, as for the improvement in terms of the extent of inflammatory cells' infiltration, degree of alveolar wall edema and congestion, and structural intactness of alveolar and bronchial lumens, each of the compositions of naringin and levocetirizine hydrochloride is obviously better than the cofetol, naringin, and levocetirizine hydrochloride.

### Example 4:

40 g of naringin and 2 g of levocetirizine hydrochloride were weighed. The 2 g of levocetirizine hydrochloride was mixed uniformly with 76 g of starch, then with naringin, and then with 2 g of finely divided silica gel, and filled into 1000 capsules, to obtain a capsule preparation.

### Example 5:

40 g of naringin and 2 g of levocetirizine hydrochloride were weighed, mixed uniformly with 76 g of starch and then with 2 g of finely divided silica gel, and filled into 1000 capsules, to obtain a capsule preparation.

### Example 6:

40 g of naringin and 2 g of levocetirizine hydrochloride were weighed. The 2 g of levocetirizine hydrochloride was mixed uniformly with 156 g of starch and then with naringin, and wet granulated. The granules were dried, mixed uniformly with 2 g of finely divided silica gel, and tabletted into 1000 tablets, to obtain a tablet preparation.

### Example 7:

40 g of naringin and 2 g of levocetirizine hydrochloride were weighed. The 2 g of levocetirizine hydrochloride was mixed uniformly with 28 g of dextrin, then with naringin, 48 g of dextrin and 2 g of finely divided silica gel in sequence, and filled into 1000 capsules, to obtain a capsule preparation.

### Example 8:

40 g of naringin and 2 g of levocetirizine hydrochloride were weighed. The 2 g of levocetirizine hydrochloride was mixed uniformly with 38 g of lactose, then with naringin and then with 118 g of starch, and wet granulated. The granules were dried, mixed uniformly with 2 g of finely divided silica gel, and tabletted into 1000 tablets, to obtain a tablet preparation.

## Claims

1. A pharmaceutical composition of naringin, comprising 27.5 to 275 mg of naringin, and 1.25 to 12.5 mg of levocetirizine hydrochloride.

2. The pharmaceutical composition according to claim 1, wherein the weight ratio of naringin to levocetirizine hydrochloride is 20:1.

3. The pharmaceutical composition according to claim 1 or 2, comprising 40 mg of naringin and 2 mg of levocetirizine hydrochloride.

4. A clinically acceptable preparation made with the pharmaceutical composition according to any one of claims 1 to 3.

5. The clinically acceptable preparation according to claim 4, which is a tablet, a capsule, an aqueous solution, or an aerosol.

6. The clinically acceptable preparation according to claim 5, wherein it comprises an adjuvant, the said adjuvant being starch, lactose, mannitol, calcium hydrophosphate, carboxymethyl starch or a salt or a substituted derivate thereof, dextrin, chitosan, polyvinyl pyrrolidone, cellulose or a derivate thereof, or polyethylene glycol.

## Patentansprüche

1. Pharmazeutische Zusammensetzung aus Naringin, umfassend 27,5 bis 275 mg Naringin und 1,25 bis 12,5 mg Levocetirizinhydrochlorid.

2. Pharmazeutische Zusammensetzung nach Anspruch 1, wobei das Gewichtsverhältnis von Naringin zu Levocetirizinhydrochlorid 20:1 beträgt.

3. Pharmazeutische Zusammensetzung nach Anspruch 1 oder 2, umfassend 40 mg Naringin und 2 mg Levocetirizinhydrochlorid.

4. Klinisch verträgliches Präparat, hergestellt aus der pharmazeutischen Zusammensetzung nach irgendeinem der Ansprüche 1 bis 3.

5. Klinisch verträgliches Präparat nach Anspruch 4, das eine Tablette, eine Kapsel, eine wässrige Lösung oder ein Aerosol ist.

6. Klinisch verträgliches Präparat nach Anspruch 5, **dadurch gekennzeichnet, dass** der Hilfsstoff Stärke, Lactose, Mannitol, Calciumhydrophosphat, Carboxymethylstärke oder ein Salz oder ein substituiertes Derivat davon, Dextrin, Chitosan, Polyvinylpyrrolidon, Cellulose oder ein Derivat davon, oder Polyethylenglykol ist.

## Revendications

1. Composition pharmaceutique à base de naringine comprenant de 27,5 à 275 mg de naringine et de 1,25 à 12,5 mg de chlorhydrate de lévocétirizine.

2. Composition pharmaceutique selon la revendication 1, dans laquelle le ratio entre la naringine et le chlorhydrate de lévocétirizine est de 20:1.

3. Composition pharmaceutique selon la revendication 1 ou 2, comprenant 40 mg de naringine et 2 mg de chlorhydrate de lévocétirizine.

4. Préparation cliniquement acceptable réalisée à l'aide de la composition pharmaceutique selon l'une quelconque des revendications 1 à 3.

5. Préparation cliniquement acceptable selon la revendication 4, qui est un comprimé, une capsule, une solution aqueuse ou un aérosol.

6. Préparation cliniquement acceptable selon la revendication 5, comprenant un adjuvant, ledit adjuvant étant de l'amidon, du lactose, du mannitol, de l'hydrophosphate de calcium, du carboxyméthylamidon ou un sel ou un dérivé substitué de ces derniers, de la dextrine, du chitosane, de la polyvinylpyrrolidone, un dérivé de ces derniers, ou du polyéthylène glycol.
